# EUROPÄISCHE PATENTSCHRIFT

(11) **EP 2 069 767 B1**
(45) Veröffentlichungstag und Bekanntmachung des Hinweises auf die Patenterteilung: **18.06.2014**
(21) Anmeldenummer: 07820593.7
(22) Anmeldetag: 26.09.2007
(51) Int. Cl.: G01N 23/04, H01J 35/08, H05G 1/04, A61B 6/03

(54) **RÖNTGENSYSTEM UND VERFAHREN ZUR TOMOSYNTHESEABTASTUNG**
X-RAY SYSTEM AND METHOD FOR TOMOSYNTHETIC SCANNING
SYSTÈME DE RADIOGRAPHIE ET PROCÉDÉ DE BALAYAGE DE TOMOSYNTHÈSE

(30) Priorität: 29.09.2006 DE 102006046741
(43) Veröffentlichungstag der Anmeldung: 17.06.2009
(73) Patentinhaber: Siemens Aktiengesellschaft, 80333 München (DE)
(72) Erfinder: MERTELMEIER, Thomas, 91058 Erlangen (DE)
(86) Internationale Anmeldenummer: PCT/EP2007/060198
(87) Internationale Veröffentlichungsnummer: WO 2008/037731

(56) Entgegenhaltungen:
- DE-A1- 3 426 934
- US-A- 4 662 379
- BISSONNETTE, HANSROUL, MASSON, SAVARD, CADIEUX, WARMOES, GRAVEL, AGOPYAN, POLISCHUK, HAERER, MERTELMEIER, LO, CHEN, DOBBINS ET.AL.: "Digital breast tomosynthesis using an amorphous selenium flat panel detector" PROCEEDINGS OF SPIE, Bd. 5745, 2005, Seiten 529-540, XP002471547
- SIEMENS MEDICAL: "MAMMOMAT Novation DR Puesto de trabajo digital para screening y diagnóstico mamográfico" SIEMENS, Mai 2006 (2006-05), XP002471548
- ANN-KATHERINE CARTON, JINGJING LI, MICHAEL ALBERT, SARA CHEN, ANDREW D.A. MAIDMENT: "Quantification for contrast-enhanced digital breast tomosynthesis" PROCEEDINGS OF SPIE, Bd. 6142, 2. März 2006 (2006-03-02), XP002471054

## Beschreibung

Die Erfindung betrifft ein Röntgensystem und ein Verfahren zur Tomosyntheseabtastung eines Objekts.

3D-bildgebende Verfahren setzten sich in der Medizintechnik immer mehr durch. Ein aufwendiges und teures Verfahren ist hierbei z.B. die Computertomographie (CT), bei welcher eine Röntgenröhre und ein ihr gegenüberliegender Detektor einen Patienten als Untersuchungsobjekt umkreisen, um hierbei Schichtaufnahmen des Patienten zu erzeugen. Aus den Schichtaufnahmen wird anschließend ein gesamtes 3D-Volumen rekonstruiert.

Ein deutlich unaufwendigeres 3D-Bildgebungsverfahren ist die digitale Tomosynthese, welche sich insbesondere im Zusammenhang mit der Mammographie in Entwicklung befindet. Im Gegensatz zur CT wird hierbei eine Röntgenquelle nur innerhalb eines beschränkten Winkelbereiches, z.B. ausgehend von einer Mittelposition um ± 20°C, um ein Untersuchungsobjekt als Zentrum der Bewegung verschwenkt. Ein Röntgendetektor zum Empfang der Röntgenstrahlen bleibt hierbei im Wesentlichen ortsfest, d.h. tatsächlich ortsfest oder er wird lediglich leicht verkippt, z.B. um der rotierenden Röntgenröhre zumindest gemäß des Einstrahlwinkels zu folgen. Die Anfertigung einer Vielzahl von Röntgenbildern aus jeweils verschiedenen Winkeln innerhalb des zu verschwenkenden Winkelbereiches wird als Tomosyntheseabtastung bezeichnet.

Da die Tomosynthese ein möglichst kostengünstiges und unaufwendiges Verfahren sein soll, werden hierbei, z.B. im Vergleich zur CT, deutlich einfachere, und damit weniger belastbare Röntgenröhren verwendet. Somit ist die Auslösegeschwindigkeit der Röntgenröhren begrenzt und eine 50°-Tomosyntheseabtastung bzw. Scan, d.h. Verschwenkung der Röntgenröhre in einem Winkelbereich von 50° zur Erzeugung der Vielzahl von Röntgenbildern bzw. Projektionsaufnahmen aus verschiedenen Blickrichtungen dauert heute zwischen zehn Sekunden und einer Minute.

Ein Röntgentomosynthesesystem und -verfahren mit verschwenkbarer Quelle und 2D-Flachdetektor ist beispielsweise aus M. Bissonnette et al., "Digital breast tomosynthesis using an amorphous selenium flat panel detector", proceedings of SPIE Vol. 5745, 2005, S. 529-540, bekannt.

Bekannt ist auch die so genannte Dual-Energy-Methode, bei welcher das Objekt aus jeder Blickrichtung zweimal mit Röntgenstrahlung zweier verschiedener Energien, bestrahlt wird. Hierbei ist bekannt, entweder zwei komplette Scans mit jeweils einer Energie auszuführen, d.h. die Energiestufe der Röntgenröhre zwischen den beiden Scans einmal umzuschalten oder einen einzigen Scan auszuführen und in jeder Winkelposition d.h. für jede Projektionsaufnahme die Röntgenröhre jeweils zwischen den beiden Energien umzuschalten. Letzteres bedeute eine große Belastung für die Röntgenröhre auch z.B. in mechanischer Hinsicht, da sowohl der Strahlerzeuger zwischen beiden Strahlqualitäten als auch die Vorfilterung (Einschwenken mechanischer Filter) umgeschaltet wird.

Die Scangeschwindigkeit wird heute in der Regel durch die Röntgenröhre begrenzt, da z.B. Detektoren mit schneller Auslesbarkeit (z.B. 30 Bilder pro sek.) verwendet werden. Bei solchen hohen Bildraten kann es aber zu thermischen Problemen in der Röntgenröhre, nicht nur in der Anode sondern auch an der Kathode kommen. Die thermischen Probleme rühren vom Abtransport der Wärme von der Anode der Röntgenröhre, da dort weniger als 1% der zugeführten Energie in Röntgenstrahlung und mehr als 99% in Wärme ungesetzt werden. Im Gegensatz zur CT mit einem Fokusdurchmesser von 1mm auf der Anode wird bei der Mammographie wegen der höheren Auflösung z.B. mit einem 0,3mm Fokus gearbeitet, was eine wesentlich höhere Energiedichte und damit deutlich größere thermische Problematik in der Röntgenröhre bedingt.

Aufgabe der vorliegenden Erfindung ist es, ein verbessertes Röntgensystem und ein verbessertes Verfahren zur Tomosyntheseabtastung anzugeben.

Die Aufgabe wird gelöst durch ein Röntgensystem zur Tomosyntheseabtastung eines Objekts mit einer Röntgenquelle, welche Röntgenstrahlung zur Durchstrahlung des Objekts aussendet und während der Tomosyntheseabtastung relativ zum Objekt verschwenkt wird. Das Röntgensystem umfasst einen während der Tomosyntheseabtastung relativ zum Objekt im Wesentlichen ortsfesten 2D-Röntgendetektor zum Empfang der Röntgenstrahlung. Erfindungsgemäß weist die Röntgenquelle mindestens zwei Strahlquellen auf, welche bezüglich ihrer Strahlrichtungen zum Objekt hin nebeneinander angeordnet und unabhängig voneinander auslösbar sind. Die Strahlquellen sind also so angeordnet, dass sie Röntgenstrahlen zum Objekt und zum 2D-Röntgendetektor hin auf verschiedenen Strahlbahnen aussenden, welche sich im Bereich des Objekts bzw. des 2D-Röntgendetektors schneiden. Der Versatz zwischen den Quellen beträgt erfindungsgemäß zumindest annähernd ein halbes Winkelinkrement.

Da die Strahlquellen unabhängig voneinander auslösbar sind, können diese zeitlich nacheinander ausgelöst werden. Durch die entsprechend wechselweise Auslösung wird die thermische Belastung für jede einzelne Strahlquelle bei gleicher Strahlleistung bzw. Bildrate des Röntgensystems halbiert.

Durch die Verwendung von mindestens zwei Strahlquellen muss für die gleiche Anzahl von Röntgenaufnahmen bzw. Abgabe von gleicher Röntgendosis während einer Tomosyntheseabtastung jede Strahlquelle nur einen Teil der Röntgenstrahlung, bei zwei Strahlquellen z.B. die Hälfte, gegenüber einem herkömmlichen Tomosyntheseröntgensystem mit einer Strahlquelle abgeben. Die Zahl der Projektionen, also Röntgenaufnahmen während der Tomosyntheseabtastung in einer gegebenen Scanzeit erzeugt werden können, erhöht sich. Bei einem entsprechend ausreichend schnellen Detektor kann daher die Scangeschwindigkeit, also die Zeit zur kompletten Tomosyntheseabtastung deutlich reduziert werden, wenn bisher die Röntgenauslösung der kritische Zeitfaktor war. Da jede Strahlquelle weniger Strahlung abgeben muss, sinkt die thermische Belastung der Strahlquellen gegenüber einer einzigen Strahlquelle ebenfalls. Die gesamte Strahlleistung des Röntgensystems erhöht sich ebenfalls mit der Anzahl von Quellen.

Somit ergibt sich eine thermische Entlastung der jeweiligen Strahlquelle bei insgesamt gleich bleibender Strahlungsleistung des Röntgensystems oder eine entsprechend vervielfachte Strahlleistung des Röntgensystems bei gleicher thermischer Belastung der Strahlquellen.

Die Strahlquellen können in einem gemeinsamen Gehäuse der Röntgenquelle angeordnet sein. Für die Tomosyntheseabtastung können dann alle Strahlquellen gleichmäßig bzw. gleichzeitig wie bisher um das Objekt verschwenkt werden. Der konstruktive Aufwand bzw. die entsprechende Ausgestaltung eines bisherigen Röntgensystems ändert sich kaum, nämlich lediglich in Bezug auf das Innenleben des Gehäuses der Röntgenquelle. Die Röntgenröhren bzw. Strahlquellen werden dabei abwechselnd bepulst, also ausgelöst.

Die Strahlquellen können in einer einzigen Röntgenröhre angeordnet sein. Bekannte Röntgensysteme sind hierdurch besonders einfach in erfindungsgemäße Röntgensysteme umzurüsten, die Leistungsbeschaltung muss auch bei einem erfindungsgemäßen Röntgensystem lediglich für eine einzige Röntgenröhre ausgelegt sein.

Im Falle einer einzigen Röntgenröhre mit mehreren Strahlquellen können für verschiedene Strahlquellen verschiedene Anoden in der Röntgenröhre enthalten sein. Für jede Strahlquelle existiert so z.B. eine eigene Anode mit einem jeweils einzigen Brennfleck. Die Anoden können hierbei bereits jeweils speziell für eine bestimmte Art von Röntgenstrahlung ausgelegt sein, so können beispielsweise beide Strahlquellen für jeweils unterschiedliche Röntgenspektren ausgebildet sein.

Alternativ können jedoch auch verschiedene Strahlquellen als verschiedene Brennflecke auf einer einzigen Anode angeordnet sein. Eine entsprechende Röntgenröhre mit einer einzigen Anode ist einfacher und unaufwendiger auszuführen.

Wie bereits erwähnt, können verschiedene Strahlquellen zur Aussendung von Röntgenstrahlen verschiedener Energie konfigurierbar sein. Zur Durchführung der Dual-Energy-Methode kann also jede Strahlquelle fest auf eine bestimmte Energiestufe der Röntgenleistung eingestellt sein. Die einzelne Strahlquelle muss so nicht permanent zwischen verschiedenen Energien umgeschaltet werden. Dies bedeutet wesentlich weniger Belastung für die Strahlquellen. In Verbindung mit einem energieauflösenden Detektor, können sogar beide Strahlquellen gleichzeitig ausgelöst werden und vom Detektor jeweils gleichzeitig zwei Röntgenbilder bzw. Projektionsaufnahmen mit verschiedener Strahlenergie aufgenommen werden. Die Scanzeit für eine Dual-Energy-Tomosyntheseabtastung reduziert sich hierdurch deutlich. Natürlich ist auch wie bisher eine Umschaltung der Strahlquellen an einem Ort denkbar, so dass ein und dieselbe Projektionsaufnahme mit unterschiedlichen Energien ausgeführt wird.

Während einer Tomosyntheseabtastung wird die Röntgenquelle in bestimmten festgelegten Winkelinkrementen relativ zum Objekt verschwenkt und bei jedem Winkelinkrement ein Röntgenbild erzeugt. Der Abstand zwischen zwei Strahlquellen kann so gewählt sein, dass er dem Winkelinkrement bzw. einem Vielfachen dessen entspricht. Aus einer Position der Röntgenquelle heraus können so durch Auslösung beider Strahlquellen zwei Projektionsaufnahmen für die Tomosyntheseabtastung erzeugt werden. Erst dann muss die Röntgenquelle wieder um ein oder mehrere Winkelinkremente verschoben werden. Sind die Strahlquellen beispielsweise um eine einziges Winkelinkrement versetzt, wird die Röntgenquelle anschließend um das doppelte Winkelinkrement versetzt und wieder zwei Röntgenaufnahmen im Abstand eines einzigen winkelinkrements von den verschiedenen Strahlquellen erzeugt. Die Schrittanzahl für die Verschwenkung der Röntgenquelle reduziert sich somit deutlich gegenüber einem bekannten Röntgensystem.

Während der gesamten Tomosyntheseabtastung wird die Röntgenquelle über einen Gesamtwinkelbereich, z.B. 50°, relativ zum Objekt verschwenkt. Z.B. zwei Strahlquellen können auch so angeordnet werden, dass deren Abstand, bzw. der Winkel zwischen den jeweiligen Strahlachsen etwa dem halben Winkelbereich entspricht. Die gesamte Röntgenquelle muss zur Abdeckung des Winkelbereiches dann nur um den halben Winkelbereich verschwenkt werden, wobei jede Strahlquelle jeweils die Hälfte des Winkelbereichs (bzw. etwas mehr wegen einer Überlappung) überstreicht.

Das erfindungsgemäße Röntgensystem eignet sich insbesondere als Röntgensystem in einer Mammographieanlage, in welcher als Objekt eine Brust eines Patienten tomosynthetisch abgetastet und anschließend im Rahmen einer Tomosynthese rekonstruiert wird.

Hinsichtlich des Verfahrens wird die Aufgabe gelöst durch ein Verfahren zur Tomosyntheseabtastung eines Objekts bei dem Röntgenstrahlung zur Durchstrahlung eines Objekts von einer Röntgenquelle ausgesandt wird, wobei die Röntgenquelle während der Tomosyntheseabtastung relativ zum Objekt verschwenkt wird. Die Röntgenstrahlung wird von einem während der Tomosyntheseabtastung bezüglich der Röntgenquelle im Wesentlichen ortsfesten Röntgendetektor empfangen. Erfindungsgemäß wird die Röntgenstrahlung von mindestens zwei, bezüglich ihrer Strahlrichtungen zum Objekt hin nebeneinander angeordneten unabhängig voneinander auslösbaren Strahlquellen in der Röntgenquelle ausgesandt. Die Röntgenquelle wird während der Tomosyntheseabtastung über einen Winkelbereich relativ zum Objekt verschwenkt, wobei zwei Strahlquellen Röntgenstrahlung in Strahlrichtungen aussenden, die zueinander zumindest annähernd dem halben Winkelbereich entsprechend geneigt sind.

Das erfindungsgemäße Verfahren und die sich daraus ergebenden Vorteile wurden bereits im Zusammenhang mit dem erfindungsgemäßen Röntgensystem erläutert. Weitere vorteilhafte Ausgestaltungen des Verfahrens wurden ebenfalls bereits im Zusammenhang mit dem erfindungsgemäßen Röntgensystem erläutert.

Für eine weitere Beschreibung der Erfindung wird auf die Ausführungsbeispiele der Zeichnungen verwiesen. Es zeigen, jeweils in einer schematischen Prinzipskizze:
- Fig. 1: ein Röntgensystem zur Tomosyntheseabtastung in der Mammographie mit einer Röntgenquelle mit zwei Strahlquellen,
- Fig. 2: ein alternatives Röntgensystem mit zwei Röntgenquellen mit je einer Strahlquelle.

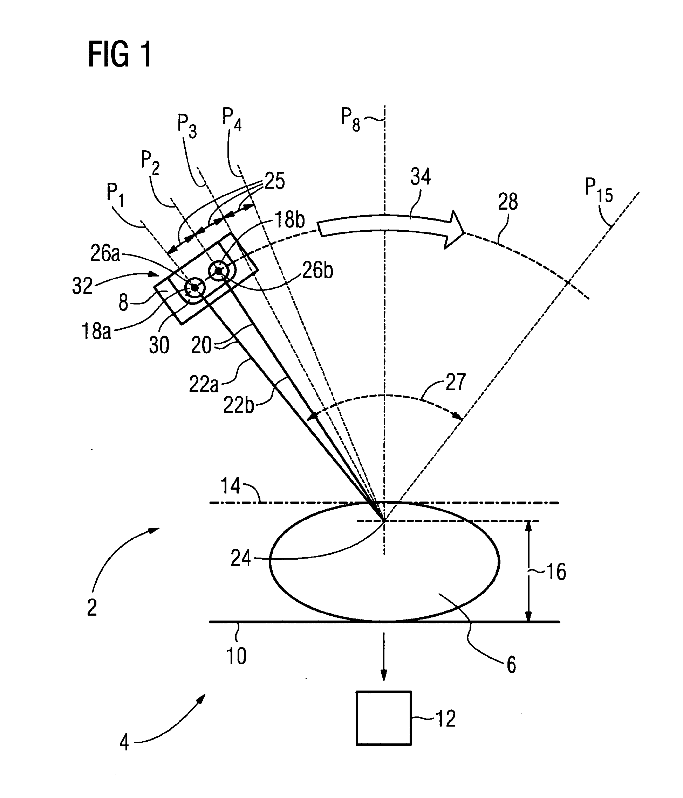

Fig. 1 zeigt ein Röntgensystem 2 zur Tomosyntheseabtastung einer in einer Haltevorrichtung 4 fixierten weiblichen Brust 6. Das Röntgensystem 2 umfasst eine Röntgenquelle 8 und einen 2D-Flachdetektor 10 zur Aufnahme von 2D-Röntgenbildern 12. Der Flachdetektor 10 dient außerdem als erste Kompressionsplatte der Haltevorrichtung 4, welche eine zweite Kompressionsplatte 14 umfasst, zwischen welchen die Brust 6 in Richtung des Doppelpfeils 16 komprimiert ist.

Die Röntgenquelle 8 umfasst zwei Strahlquellen 18a,b welche jeweils Röntgenstrahlung 20 in Richtung einer Strahlmittelachse 22a,b aussenden. Die Strahlmittelachsen 22a,b schneiden sich in einem Punkt einer Schwenkachse 24, welche in Fig. 1 senkrecht zur Zeichenebene und damit parallel zum Flachdetektor 10 bzw. der Kompressionsplatte 14 im Inneren der Brust 6 verläuft.

Um diese Schwenkachse 24 ist auch die gesamte Röntgenquelle 8 verschwenkbar, so dass jeweilige Fokuspunkte 26a,b der Strahlquellen 18a,b auf einer kreisabschnittförmigen Linie 28 laufen. In Fig. 1 liegen beide Fokuspunkte 26a,b als zwei Brennflecke auf einer einzigen Anode 30 einer Röntgenröhre 32 (nur schematisch dargestellt).

Zur tomosynthetischen Darstellung der Brust 6 müssen zunächst eine Vielzahl von Röntgenbildern 12 von dieser erzeugt werden. Dies geschieht bei der Tomosyntheseabtastung, indem jeweils Röntgenstrahlung 20 entlang einer der Projektionslinie bzw. -richtung P₁ bis P₁₅ zum Flachdetektor 10 ausgesandt wird und für jede dieser Projektionsrichtungen ein Röntgenbild 12, also eine Projektionsaufnahme erzeugt wird. Sämtliche Projektionslinien P₁-P₁₅ sind hierbei jeweils bezüglich der Schwenkachse 24 um 5° versetzt, so dass sich bei 15 Projektionslinien insgesamt ein Verschwenkbereich 27 der Röntgenbilder 12 von 70° ergibt. Das jeweilige Winkelinkrement 25 für zwei Projektionsaufnahmen beträgt in Fig. 1 also 5°

Die Mammographie in Fig. 1 erfolgt nun so, dass die Röntgenquelle 8 in der gezeigten Position durch die beiden Fokuspunkte 26a,b die Projektionslinien P₁ und P₂ abdeckt und entlang dieser, also entlang ihrer Strahlmittelachsen 22a,b Röntgenstrahlung 20 aussendet, um zwei Röntgenbilder 12 in zeitlicher Abfolge hintereinander zu erzeugen. Anschließend wird die gesamte Röntgenquelle 8 in Richtung des Pfeils 34, also in Scan- bzw. Tomosyntheseabtastrichtung um zwei Winkelinkremente, also 10° um die Schwenkachse 24 verschwenkt, so dass die Fokuspunkte 26a, b auf den Projektionslinien P₃ und P₄ zum liegen kommen. Aus dieser Position heraus werden wiederum die Strahlquellen 18a,b zeitlich hintereinander ausgelöst, um zwei weitere Röntgenbilder 12 zu erzeugen. Dieser Vorgang wird solange wiederholt, bis der Fokuspunkt 26b die Projektionslinie P₁₅ erreicht hat, um das fünfzehnte und somit letzte Röntgenbild 12 zu erzeugen.

Alternativ ist auch eine kontinuierliche Bewegung der Strahlquellen 18a,b möglich, wobei diese dann an den Projektionslinien P₁₋₁₅, also bei deren Passieren gepulst werden, also pulsförmig Röntgenstrahlung 20 erzeugt wird.

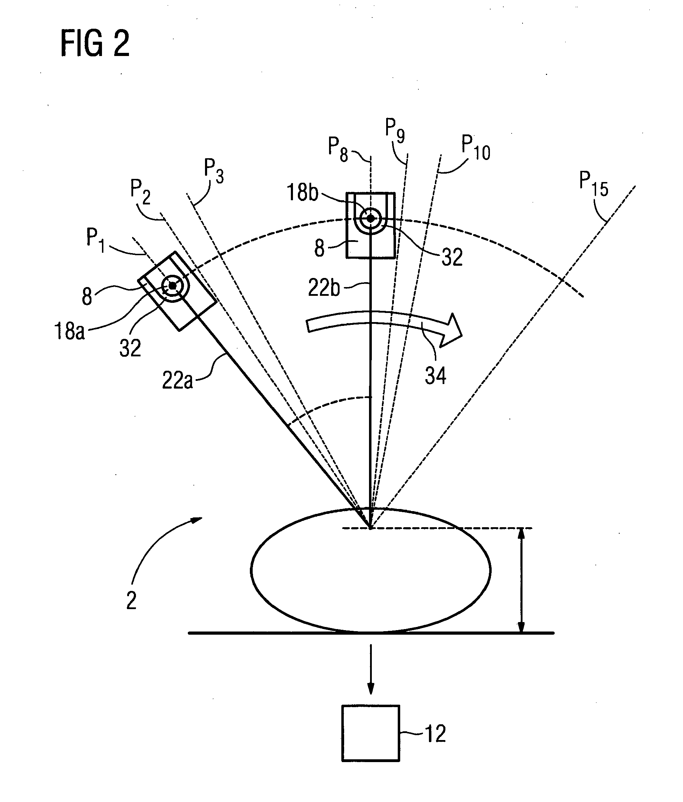

Fig. 2 zeigt eine alternative Ausführungsform eines Röntgensystems 2, bei der beide Strahlquellen 18a,b in einer jeweils separaten Röntgenquelle 8 angebracht sind. In jeder Röntgenquelle 8 befindet sich also eine Röntgenröhre 32. Gegenüber Fig. 1 sind in Fig. 2 die Strahlquellen 18a,b bezüglich ihrer Strahlmittenachsen 22a,b wesentlich weiter voneinander beabstandet. Diese schließen nämlich insgesamt einen Winkel von sieben Winkelinkrementen, also 35° ein. In der in Fig. 2 dargestellten Situation fällt daher die Strahlmittelachse 22a mit der Projektionslinie P₁ und die Strahlmittelachse 22b mit der Projektionslinie P₈ zusammen. Der relative Winkel zwischen beiden Strahlmittelachsen 22a,b ist, wie in Fig. 1 fest eingestellt und somit nicht veränderbar während der Tomosyntheseabtastung, z.B. durch Montage der beiden Röntgenquellen auf einen gemeinsamen, nicht dargestellten Schwenkarm.

Auch in Fig. 2 werden zunächst die Strahlquellen 18a,b zeitlich hintereinander ausgelöst, wodurch zwei Röntgenbilder 12 entstehen. Anschließend werden die beiden Röntgenquellen 8 synchron, jedoch lediglich um ein Winkelinkrement, also 5°, also halb so weit wie in Fig. 1 verschwenkt. Die Strahlmittelachsen 22a,b fallen dann mit den Projektionslinien P₂ und P₉ zusammen. Nun werden wieder zwei Röntgenbilder 12 erzeugt und der entsprechende Vorgang solange wiederholt, bis die Strahlquelle 18b wieder auf der Projektionslinie P₁₅ liegt. Im Gegensatz zu Fig. 1 muss also jede Röntgenquelle 87 nur um 35°, also den halben Gesamtwinkelbereich zwischen den Projektionslinien P₁ und P₁₅ von 70° verschwenkt werden.

Alternativ können auch in Fig. 1 die Strahlquellen 18a,b gemäß Fig. 2 getrennt aufgebaut sein.

## Patentansprüche

1. Röntgensystem (2) zur Tomosyntheseabtastung eines Objekts (6), mit einer Röntgenstrahlung (20) zur Durchstrahlung des Objekts (6) aussendenden, während der Tomosyntheseabtastung relativ zum Objekt (6) verschwenkbaren Röntgenquelle (8), und mit einem während der Tomosyntheseabtastung relativ zum Objekt (6) im wesentlichen ortsfesten 2D-Röntgendetektor (10) zum Empfang der Röntgenstrahlung (20), wobei die Röntgenquelle (8) mindestens zwei, bezüglich ihrer Strahlrichtungen (22a,b) zum Objekt (6) hin nebeneinander angeordnete, unabhängig voneinander auslösbare Strahlquellen (18a,b) aufweist und wobei die Röntgenquelle (8) während der Tomosyntheseabtastung über einen Winkelbereich (27) relativ zum Objekt (6) verschwenkbar ist, bei dem der Abstand zwischen zwei Strahlquellen (18a,b) zumindest annähernd dem halben Winkelbereich (27) entspricht.

2. Röntgensystem (2) nach Anspruch 1, bei dem die Strahlquellen (18a,b) in einem gemeinsamen Gehäuse der Röntgenquelle (8) angeordnet sind.

3. Röntgensystem (2) nach Anspruch 1 oder 2, bei dem die Strahlquellen (18a,b) in einer einzigen Röntgenröhre (32) angeordnet sind.

4. Röntgensystem (2) nach Anspruch 3, bei dem für verschiedene Strahlquellen (18a,b) verschiedene Anoden (30) in der Röntgenröhre (32) enthalten sind.

5. Röntgensystem (2) nach Anspruch 3 oder 4, bei dem für verschiedene Strahlquellen (18a,b) verschiedene Brennflecke auf einer Anode (30) angeordnet sind.

6. Röntgensystem (2) nach einem der vorhergehenden Ansprüche, bei dem verschiedene Strahlquellen (18a,b) zur Aussendung von Röntgenstrahlung (20) verschiedener Energie konfigurierbar sind.

7. Röntgensystem (2) nach einem der vorhergehenden Ansprüche, wobei die Röntgenquelle (8) während der Tomosyntheseabtastung in festlegbaren Winkelinkrementen (25) relativ zum Objekt (6) verschwenkbar ist, bei dem der Abstand zwischen zwei Strahlquellen (18a,b) dem Winkelinkrement (25) oder einem ganzzahligen Vielfachen dessen entspricht.

8. Röntgensystem (2) nach einem der vorhergehenden Ansprüche, das ein Röntgensystem (2) in einer Mammographieanlage zur Tomosynthese einer Brust eines Patienten als Objekt (6) ist.

9. Verfahren zur Tomosyntheseabtastung eines Objekts (6), bei dem
- Röntgenstrahlung (20) zur Durchstrahlung des Objekts (6) von einer Röntgenquelle (8) ausgesandt wird, wobei die Röntgenquelle (8) während der Tomosyntheseabtastung relativ zum Objekt (6) verschwenkt wird,
- die Röntgenstrahlung (20) von einem während der Tomosyntheseabtastung bezüglich der Röntgenquelle (8) im Wesentlichen ortsfesten 2D-Röntgendetektor (10) empfangen wird,
- wobei die Röntgenstrahlung (20) von mindestens zwei, bezüglich ihrer Strahlrichtungen (22a,b) zum Objekt (6) hin nebeneinander angeordneten, unabhängig voneinander auslösbaren Strahlquellen (18a,b) in der Röntgenquelle (8) ausgesandt wird,
- wobei die Röntgenquelle (8) während der Tomosyntheseabtastung über einen Winkelbereich relativ zum Objekt (6) verschwenkt wird, bei dem zwei Strahlquellen Röntgenstrahlung in Strahlrichtungen (22a,b) aussenden, die zueinander zumindest annähernd dem halben Winkelbereich entsprechend geneigt sind.

10. Verfahren nach Anspruch 9, bei dem zwei Strahlquellen (18a,b) zeitlich nacheinander ausgelöst werden.

11. Verfahren nach Anspruch 9 oder 10, bei dem zwei Strahlquellen (18a,b) Röntgenstrahlung (20) unterschiedlicher Energie aussenden.

12. Verfahren nach Anspruch 11, bei dem zwei Strahlquellen gleichzeitig ausgelöst werden.

13. Verfahren nach einem der Ansprüche 9 bis 12, wobei die Röntgenquelle (8) in festgelegten Winkelinkrementen (25) relativ zum Objekt (6) verschwenkt wird, bei dem
- zwei Strahlquellen (18a,b) Röntgenstrahlung (20) in Strahlrichtungen (22a,b) aussenden, die zueinander dem Winkelinkrement (25) oder einem ganzzahligen Vielfachen dessen entsprechend geneigt sind.

14. Verfahren nach Anspruch 13, bei dem die Strahlquellen (18a,b) alternierend um verschiedene Winkelinkremente (25) verschwenkt werden.

15. Verfahren nach Anspruch 9 bis 14, bei dem die Strahlquellen (18a,b) in einer kontinuierlichen Bewegung um das Objekt (6) verschwenkt werden und dabei pulsförmig Röntgenstrahlung (20) aussenden.

16. Verfahren nach einem der Ansprüche 9 bis 15, das im Rahmen einer Mammographie einer Brust eines Patienten als Objekt (6) verwendet wird.

## Claims

1. X-ray system (2) for tomosynthetic scan of an object (6), comprising an x-ray source (8), which emits x-ray radiation (20) for passing through the object (6) and which can be pivoted relative to the object (6) during the tomosynthetic scan, and comprising a 2D x-ray detector (10) for receiving the x-ray radiation (20), which 2D x-ray detector is substantially stationary relative to the object (6) during the tomosynthetic scan, the x-ray source (8) having at least two beam sources (18a,b), which are arranged next to one another with respect to the beam directions (22a,b) thereof to the object (6) and which can be triggered independently of one another, and the x-ray source (8) being pivotable relative to the object (6) over an angle range (27), during the tomosynthetic scan, wherein the distance between two beam sources (18a,b) corresponds at least approximately to half the angle range (27).

2. X-ray system (2) according to Claim 1, wherein the beam sources (18a,b) are arranged in a common housing of the x-ray source (8).

3. X-ray system (2) according to Claim 1 or 2, wherein the beam sources (18a,b) are arranged in a single x-ray tube (32).

4. X-ray system (2) according to Claim 3, wherein different anodes (30) are contained in the x-ray tube (32) for different beam sources (18a,b).

5. X-ray source (2) according to Claim 3 or 4, wherein different foci are arranged on an anode (30) for different beam sources (18a,b).

6. X-ray system (2) according to one of the preceding claims, wherein different beam sources (18a,b) can be configured to emit x-ray radiation (20) with different energies.

7. X-ray system (2) according to one of the preceding claims, the x-ray source (8) being able to be pivoted relative to the object (6) at definable angle increments (25) during the tomosynthetic scan, wherein the distance between two beam sources (18a,b) corresponds to the angle increment (25) or an integer multiple thereof.

8. X-ray system (2) according to one of the preceding claims, which is an x-ray system (2) in a mammography machine for tomosynthesis of a breast of a patient as object (6).

9. Method for tomosynthetic scan of an object (6), wherein
- x-ray radiation (20) is emitted by an x-ray source (8) for passing through the object (6), the x-ray source (8) being pivoted relative to the object (6) during the tomosynthetic scan,
- the x-ray radiation (20) is received by a 2D x-ray detector (10) which is substantially stationary with respect to the x-ray source (8) during the tomosynthetic scan,
- the x-ray radiation (20) being emitted by at least two beam sources (18a,b) in the x-ray source (8), which beam sources are arranged next to one another with respect to the beam directions (22a,b) thereof to the object (6) and which can be triggered independently of one another,
- the x-ray source (8) being pivoted relative to the object (6) over an angle range during the tomosynthetic scan, wherein two beam sources emit x-ray radiation in beam directions (22a,b) which are tilted to one another in a manner corresponding at least approximately to half the angle range.

10. Method according to Claim 9, wherein two beam sources (18a,b) are triggered successively in time.

11. Method according to Claim 9 or 10, wherein two beam sources (18a,b) emit x-ray radiation (20) with different energies.

12. Method according to Claim 11, wherein two beam sources are triggered simultaneously.

13. Method according to one of Claims 9 to 12, the x-ray source (8) being pivoted in defined angle increments (25) relative to the object (6), wherein
- two beam sources (18a,b) emit x-ray radiation (20) in beam directions (22a,b) which are tilted to one another in a manner corresponding to the angle increment (25) or an integer multiple thereof.

14. Method according to Claim 13, wherein the beam sources (18a,b) are alternately pivoted about different angle increments (25).

15. Method according to Claim 9 to 14, wherein the beam sources (18a,b) are pivoted around the object (6) in a continuous movement and emit x-ray radiation (20) in a pulse-shaped manner in the process.

16. Method according to one of Claims 9 to 15, which is used within the scope of a mammography of a breast of a patient as object (6).

## Revendications

1. Système de radiographie (2) pour le balayage de tomosynthèse d'un objet (6), comprenant une source de rayons X (8) émettant des rayons X (20) pour radiographier l'objet (6) et pouvant être amenée à pivoter par rapport à l'objet (6) pendant le balayage de tomosynthèse, et un détecteur de rayons X (10) à deux dimensions essentiellement fixe par rapport à l'objet (6) pendant le balayage de tomosynthèse, destiné à recevoir les rayons X (20), la source de rayons X (8) comportant au moins deux sources de rayonnement (18a, b) pouvant être déclenchées indépendamment l'une de l'autre et placées l'une à côté de l'autre par rapport à leur direction de rayonnement (22a, b) vers l'objet (6) et la source de rayons X (8) pouvant être amenée à pivoter par rapport à l'objet (6) sur une zone angulaire (27) pendant le balayage de tomosynthèse, l'écartement entre deux sources de rayonnement (18a,b) correspondant au moins à peu près à la moitié de la zone angulaire (27).

2. Système de radiographie (2) selon la revendication 1, dans lequel les sources de rayonnement (18a,b) sont situées dans un boîtier commun de la source de rayons X (8).

3. Système de radiographie (2) selon la revendication 1 ou 2, dans lequel les sources de rayonnement (18a,b) sont situées dans un tube à rayons X (32) unique.

4. Système de radiographie (2) selon la revendication 3, dans lequel, pour des sources de rayonnement (18a,b) différentes, le tube à rayons X (32) contient des anodes différentes (30).

5. Système de radiographie (2) selon la revendication 3 ou 4, dans lequel, pour des sources de rayonnement (18a,b) différentes, des tâches focales différentes sont situées sur une anode (30).

6. Système de radiographie (2) selon l'une des revendications précédentes, dans lequel différentes sources de rayonnement (18a,b) peuvent être configurées pour émettre des rayons X (20) d'énergie différente.

7. Système de radiographie (2) selon l'une des revendications précédentes, la source de rayons X (8) pouvant être amenée à pivoter par rapport à l'objet (6) pendant le balayage de tomosynthèse selon des incréments angulaires (25) prédéterminables, l'écartement entre deux sources de rayonnement (18a,b) correspondant à l'incrément angulaire (25) ou à un multiple entier de celui-ci.

8. Système de radiographie (2) selon l'une des revendications précédentes, qui est un système de radiographie (2) dans une installation de mammographie destinée à la tomosynthèse d'un sein d'une patiente en tant qu'objet (6).

9. Procédé de balayage de tomosynthèse d'un objet (6), dans lequel
- des rayons X (20) permettant de radiographier l'objet (6) sont émis par une source de rayons X (8), la source de rayons X (8) étant amenée à pivoter par rapport à l'objet (6) pendant le balayage de tomosynthèse,
- les rayons X (20) sont reçus par un détecteur de rayons X (10) à deux dimensions essentiellement fixe par rapport à la source de rayons (8) pendant le balayage de tomosynthèse,
- les rayons X (20) étant émis par au moins deux sources de rayonnement (18a, b) contenues dans la source de rayons X (8), qui peuvent être déclenchées indépendamment l'une de l'autre et sont placées l'une à côté de l'autre par rapport à leur direction de rayonnement (22a, b) vers l'objet (6),
- la source de rayons X (8) étant amenée à pivoter par rapport à l'objet (6) sur une zone angulaire pendant le balayage de tomosynthèse, deux sources de rayonnement émettant des rayons X dans des directions de rayonnement (22a,b) qui présentent l'une par rapport à l'autre une inclinaison correspondant au moins à peu près à la moitié de la zone angulaire.

10. Procédé selon la revendication 9, dans lequel deux sources de rayonnement (18a,b) sont déclenchées l'une après l'autre dans le temps.

11. Procédé selon la revendication 9 ou 10, dans lequel deux sources de rayonnement (18a,b) émettent des rayons X (20) d'énergie différente.

12. Procédé selon la revendication 11, dans lequel deux sources de rayonnement sont déclenchées simultanément.

13. Procédé selon l'une des revendications 9 à 12, la source de rayons X (8) étant amenée à pivoter par rapport à l'objet (6) selon des incréments angulaires (25) prédéterminés,
- deux sources de rayonnement (18a,b) émettant des rayons X (20) dans des directions de rayonnement (22a,b) qui présentent l'une par rapport à l'autre une inclinaison correspondant à l'incrément angulaire (25) ou à un multiple entier de celui-ci.

14. Procédé selon la revendication 13, dans lequel les sources de rayonnement (18a,b) sont amenées à pivoter selon des incréments angulaires (25) différents en alternance.

15. Procédé selon les revendications 9 à 14, dans lequel les sources de rayonnement (18a,b) sont amenées à pivoter autour de l'objet (6) en un mouvement continu et émettent pendant ce temps des rayons X (20) sous forme de pulsations.

16. Procédé selon l'une des revendications 9 à 15, qui est utilisé dans le cadre d'une mammographie d'un sein d'une patiente en tant qu'objet (6).
